## Europäisches Patentamt
## European Patent Office
## Office européen des brevets

(11) Publication number: **0 044 340**
**B1**

## EUROPEAN PATENT SPECIFICATION

(45) Date of publication of patent specification: **23.10.85**

(21) Application number: **81900600.8**

(22) Date of filing: **27.01.81**

(86) International application number:
**PCT/US81/00116**

(87) International publication number:
**WO 81/02108 06.08.81 Gazette 81/19**

(51) Int. Cl.⁴: **A 61 L 2/06,** A 61 L 2/26,
B 65 D 81/20, F 01 B 15/00,
F 01 B 19/00, F 15 B 13/02,
F 16 K 31/126

(54) **PRESSURE AND TEMPERATURE RESPONSIVE DEVICE AND APPARATUS FOR CONTAINING ITEMS TO BE STERILIZED.**

(30) Priority: **28.01.80 US 115678**

(43) Date of publication of application:
**27.01.82 Bulletin 82/04**

(45) Publication of the grant of the patent:
**23.10.85 Bulletin 85/43**

(84) Designated Contracting States:
**AT CH DE FR GB LI LU NL SE**

(56) References cited:
FR-A-2 335 239
US-A-2 187 154
US-A-3 060 897
US-A-3 066 698
US-A-3 112 045
US-A-3 250 295
US-A-3 307 739
US-A-3 458 275
US-A-4 105 407
US-A-4 149 650
US-A-4 186 653
US-A-4 196 166
US-A-4 228 914
US-A-4 247 517
US-A-4 249 457

(73) Proprietor: **SANDERSON, Roger, S.**
**24772 Santa Clara Avenue**
**Dana Point, CA 92629 (US)**

(72) Inventor: **SANDERSON, Roger S.**
**24772 Santa Clara**
**Dana Point, CA 92629 (US)**
Inventor: **WHELCHEL, Robert C.**
**546 Tustin Avenue**
**Newport Beach, CA 92660 (US)**

(74) Representative: **Jones, Colin**
**W.P. THOMPSON & CO. Coopers Building**
**Church Street**
**Liverpool L1 3AB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 044 340 B1

## Description

The invention relates to a device which is responsive to pressure and temperature and which is useful for automatically releasing a container lid at a predetermined temperature. The device is particularly useful in connection with the sterilization and storage of medical items, such as surgical instruments.

A need exists for an improved system for sterilizing surgical instruments and other medical items in that the common method of wrapping articles in sheets, sterilizing then and then storing them while still in the sheets, is an unsatisfactory approach. Scientific studies have shown that thirty percent of the packs prepared with sheets are contaminated by bacteria at the time of use. Further, instruments in such packs using sheets are contaminated with lint.

In an earlier system (WO-79/00077), articles to be sterilized are placed in a container, and the container is placed in an autoclave with the lid open. After the articles have been sterilized, a pressure responsive actuator automatically releases the lid and allows it to fall into a closed position wherein a resilient gasket prevents further flow into the container. The actuator utilizes an expandable chamber which responds to pressure changes to produce an actuating movement. In a preferred approach, a quantity of sterilizing fluid is captured within the chamber by means of a temperature responsive valve. Further improvements have been made in connection with a production version of the system.

The present invention resides in a device responsive to pressure and temperature and comprising an expandable chamber impart defined by a flexible diaphragm and communicating with the atmosphere via a port which can be closed by a temperature-responsive valve element to capture a quantity of fluid in the chamber, which fluid is to expand the chamber and thereby flex the diaphragm when the pressure outside the diaphragm becomes less than the pressure inside the chamber, characterised in that the device includes a thin but relatively stiff plastic plate to which the diaphragm is secured to define the chamber between the plate and the diaphragm, in that said port is formed by a passage in said plate, and in that said diaphragm is in the form of a thin plastic, bellows like element.

Advantageously, the support plate may be moulded as a one-piece, relatively stiff plastic member, with a tab on its lower end for mounting on the periphery of a container base. In a preferred approach, the support plate has a pair of projections which, in use, extend beneath the edge of the container lid to hold it in open position.

Further, the support plate preferably is provided with an inlet nipple that extends into the expandable chamber to permit steam or other sterilizing fluid to enter the chamber during the sterilizing phase of the autoclave cycle. A heat shrinkable sleeve valve element surrounds the nipple to close the chamber during the sterilizing phase to capture a volume of fluid in the chamber.

As the container lid falls onto the base, it is critical that a seal be provided to prevent further flow into the container. A resilient gasket carried by the lid is formed with a lower flap having a feathered edge which engages a mating surface in the base to provide this initial seal. As a vacuum is formed in the container either by a final vacuum in an autoclave cycle or by the cooling of the residual environment in the container, the gasket is further compressed between the lid and the base. An enlarged bead on the gasket is compressed between the lid and the base to form a second seal for the container. Thus the container contents are sealed and preserved in a sterile, lint-free environment.

The invention includes apparatus for containing items to be sterilized and subsequently stored, comprising a container formed by a base and a lid co-operating with the base, and a device mounted on a peripheral flange of the base and including an element for propping the lid in an open position relative to the base and flexible means in part defining an expandable chamber effective upon expansion so that the flexible means displaces the propping element, allowing the lid to move to a closed position relative to the base, characterised in that the device includes a support member whose lower end is connected to the base flange and whose upper portion has at least one projection thereon, said projection being arranged to be normally beneath the rim of the lid so as to institute said element for propping the lid open, in that said support member is so constructed as to bias·the upper portion thereof towards the propped lid and so maintain the projection beneath the lid rim, and in that the expandable chamber is defined between the support member and the flexible means which is carried thereby, such that the lid is engaged by the device upon expansion of the chamber to displace the upper portion of the support member away from the lid and thus withdraw the projections from beneath the lid rim, thereby allowing the lid to fall to a closed position relative to the base.

For a more thorough understanding of the invention, reference will now be made to the following detailed description and accompanying drawings, in which:

Figure 1 is a perspective view of the overall container showing the lid of the container held in an open position;

Figure 2 is a side elevational view of the container of Figure 1, partially cut away;

Figure 3 is a side elevation of the container of Figure 2 after the lid has fallen into closed position;

Figure 4 is a fragmentary, perspective view of one end of the container base;

Figure 5 is a fragmentary, cross-sectional view of the container base of Figure 4 showing the lid and gasket positioned on the base, when the other end of the lid is held in an open position;

Figure 6 is a perspective, exploded view of the expandable chamber actuator and a fragment of the container base illustrating the manner in which the actuator is mounted on the base;

Figure 7 is a view like Figure 6 but with the actuator mounted on the base and the chamber expanded;

Figure 8 is a cross-sectional view of the expandable chamber showing the expanded position of the chamber in phantom lines;

Figure 9 is a cross-sectional view of the inlet valve of the expandable chamber showing the valve in closed position;

Figure 10 is a fragmentary view of the container showing the actuator in side elevation on the container base and holding the lid open showing the lid, base and gasket in cross-section;

Figure 11 is a cross-sectional view of the gasket free form;

Figure 12 is a cross-sectional view of the gasket mounted on a portion of the lid, also shown in cross-section;

Figure 13 is a cross-sectional view of a portion of the lid, gasket and base with the lid in closed position on the base;

Figure 14 is a perspective view of the relief valve for the sterilizing container;

Figure 15 is a cross-sectional view of the valve of Figure 14 installed in an opening in the container lid; and

Figure 15a shows the valve with a vacuum in the container;

Figure 16 is a perspective view on the upper side of the valve of Figure 14 showing the valve held in open position; and

Figure 17 is a side view partially sectionalized showing a support cone in the container basket illustrating the relation between the cone, basket cover, and the container lid.

Detailed Description

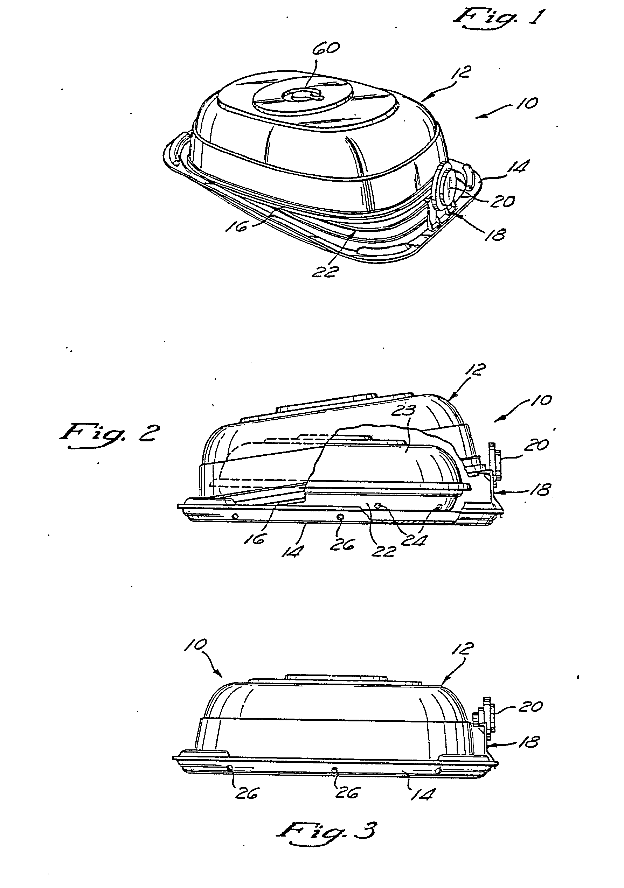

Referring now to Figures 1 and 2, there is shown a container 10 having access means or a lid 12 closing the open upper side of a base 14, with a gasket 16 carried by the lid and extending between the base and the lid. The container illustrated has a generally oval or racetrack configuration with the container lid having a somewhat dome-shape for strength purposes. Other configurations, such as circular, could also be employed. The upper portion of the lid is shaped to mate with recesses in the container base to facilitate stacking of the containers.

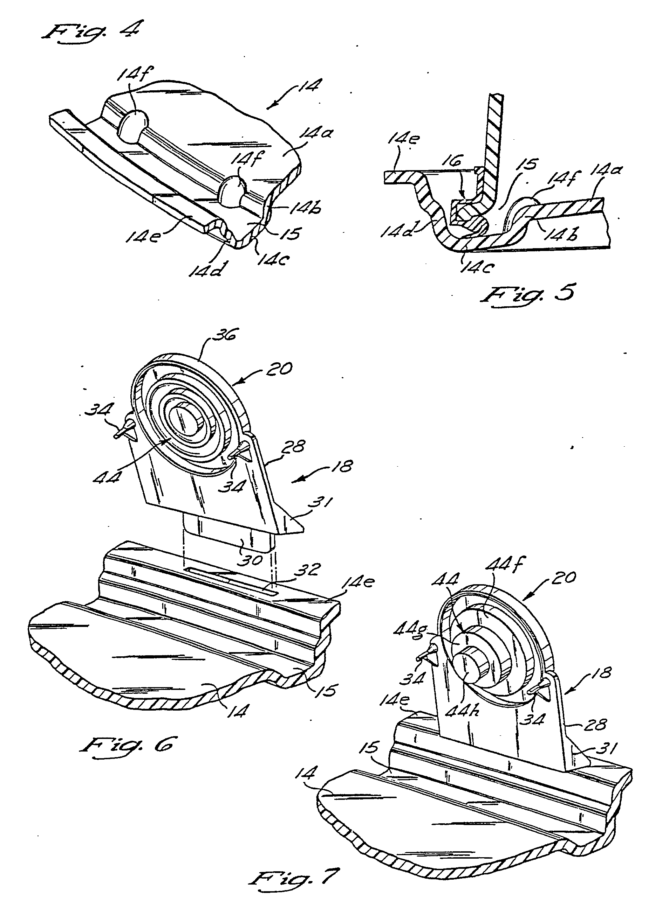

One end of the lid 12 is held open by an actuator 18 which is mounted on the base 14. The actuator includes an inflatable chamber 20 which operates to release the lid at a desired point in an autoclave sterilizing cycle, allowing the lid to drop to the position shown in Figure 3. Figure 2 also shows a basket 22 and cover 23 within the container for holding items to be sterilized and to add support to the container when a vacuum exists in it. One suitable material for the container is polysulfone which is sold by Union Carbide Company.

The basket includes a plurality of holes 24 spaced around the lower side wall of the basket, to permit sterilizing fluid to circulate and to allow air to escape. Also provided are a plurality of drain holes (not shown) in the basket bottom wall to permit condensation to drain from the basket. Referring to Figure 5 it may be seen that the container base 14 includes a bottom wall 14a which slopes downwardly and outwardly to a shoulder 14b leading to a peripheral groove 15. The bottom wall 14c of the groove 15 also slopes slightly downwardly in the outward direction to ensure that condensation will flow through the drain holes in the basket and drain holes 26 in the base, shown in Figure 2. The periphery of the base includes an upwardly and outwardly sloping wall 14d terminating in a generally horizontal flange 14e.

The base is formed with protuberances 14f to help guide the lid into its proper position when it is being installed, as shown in Figure 1, and to help prevent the lid from being improperly positioned on the base. Figure 5 shows the condition of the gasket, lid and base on the left end of the container when the right end of the container is held in open position as shown in Figure 1.

Referring to Figures 6 and 7, it may be seen that the actuator 18 includes a plate-like member 28 having on its lower end a tab 30 which snaps into a slot 32 formed in the base flange 14e on the right end of the container as shown in Figure 1. The actuator 18 further includes a pair of projections or posts 34 which extend outwardly from the plate 28. The plate 28 is preferably formed as a one-piece plastic member formed in a single molding operation.

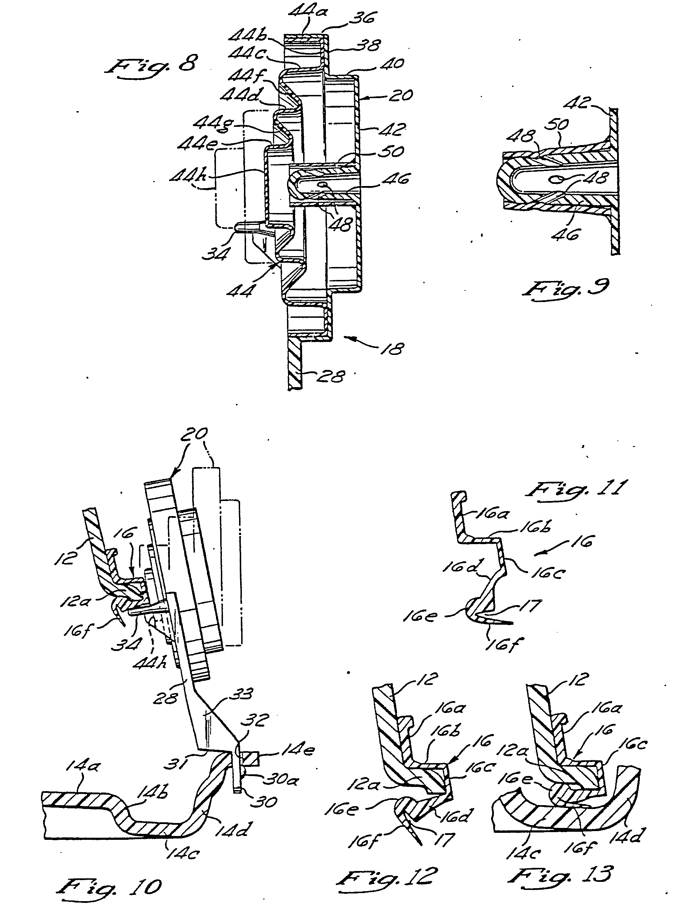

Referring to Figure 10, it may be seen that the tab 30 on the plate 28 includes one or more detents 30a which require the tab 30 to be snapped into position through the slot 32 in the flange 14e. This attachment coupled with the sides of the slot 32 in the somewhat horizontal wall 31 on the plate 28 support the actuator plate in a position extending upwardly approximately as shown in somewhat cantilever fashion. As can be seen the post-like projections 34 on the support plate 28 cooperate with the lid 12 by extending beneath the gasket 16 on the lid. Actually, with the lid removed but with the support plate mounted on the base as shown in Figure 10, the upper end of the support plate will move further than shown in Figure 10 towards the lid 12. This ensures that the lid is securely supported when the container is placed in an autoclave. Note from Figure 10 that the support plate 28 extends inwardly towards the lid at its upper end as opposed to being completely vertical.

The support plate 28 provides a number of different characteristics. First, it should be sufficiently stiff and strong to support the lid and to provide the necessary reliability. In addition it should be relatively inexpensive so that it may be disposable. Molding the support plate 28 in a single operation with its multiple functions greatly contributes to this. In order to minimize the amount of material required and yet attain the necessary stiffness and flexibility, the plate may be formed with a plurality of gussets 33 extending between the horizontal wall 31 and the approximately vertical portions of the plate. Similarly, the edges of the upright portion may be thickened or ribbed to provide the necessary strength.

Referring to Figure 8 as well as to Figure 6, it may be seen that the expandable chamber 20 is partially formed by a portion of the support plate 28. More specifically, the upper portion of the support plate is molded with a circular recess of two different diameters. The outer portion includes a cylindrical wall 36 and an annular wall 38, which is further connected to a smaller diameter cylindrical wall 40 which is joined to a circular end wall 42. Together these walls form a cup-shaped recess.

The expandable portion of the chamber 20 is formed by separate bellows-like element 44 molded of a plastic material similar to that from which the plate 28 is molded but being of thinner cross-section and being more flexible. As can be seen the diaphragm 44 includes an outer cylindrical wall 44a connected to an annular wall 44b which mate with the walls 36 and 38 on the plate 28. These walls are joined by suitable means to form the expandible chamber 20. The diaphragm 44 further includes short cylindrical wall sections 44c, 44d and 44e with progressively smaller diameters joined by connecting wall sections 44f and 44g. A central circular wall section 44h connected to the cylindrical wall 44e forms an end wall of the chamber. As can be seen from the phantom lines in Figure 8, the diaphragm 44 assumes the position indicated when the chamber is fully expanded. Note that the cylindrical walls maintain their approximate configurations but are moved outwardly due to the flexibility of the connecting annular wall sections 44f and 44g.

The support plate 28 includes a tubular portion or nipple 46 which is formed integral with the wall 42 and projects into the chamber 20. The inner end of the nipple is closed but a plurality of ports 48 in the side wall of the nipple connect the chamber 20 to the space around it. The nipple 46 tapers slightly inwardly to facilitate a single molding operation for the plate 28 and the ports 48 are formed at an angle to the side wall of the nipple so that the ports may also be made during the molding operation. That is, the mold structure forming the interior of the nipple and the ports may be withdrawn from the back side of the plate 28 at the completion of a molding operation. The material forming the plate is somewhat flexible to permit such withdrawal.

Positioned loosely over the nipple 46 is a cylindrical sleeve 50 made of heat-shrinkable material. Although the sleeve is relatively confined within the chamber, it may be more positively secured to the end plate 42 by a small amount of adhesive on the end of the sleeve.

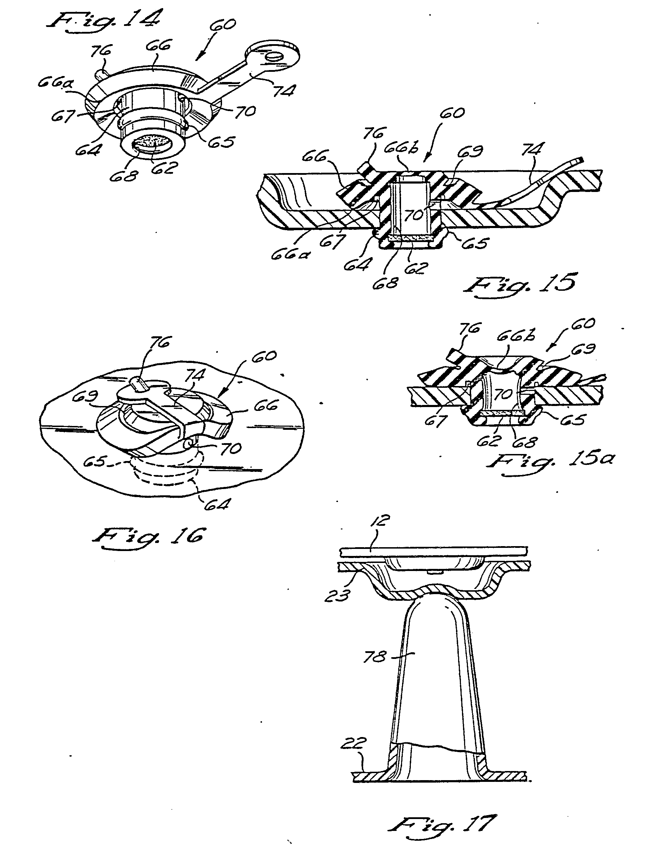

Referring to Figure 11, the gasket 16 provides a critical function requiring very flexible resilient material formed in a specific design. The gasket 16 includes an upper generally cylindrical portion 16a having on its upper edge a thickened bead adding to strength. The lower end of the portion 16a is connected to the upper leg 16b with a central section which takes a generally U-shape when installed on the lid. In addition to the leg 16b, this includes an annular wall 16c and a lower leg 16d, which in its free form shape extends somewhat downwardly. The outer end of the leg 16d is thickened to form a sealing bead 16e which leads to a thin flap 16f which tapers to a feathered lower edge. Note that there is a rather acute angle 17 between the flap 16f and the back side of the bead portion 16e.

As may be seen from Figure 12, the gasket 16 mounts on an outwardly extending flange 12a formed on the lower end of the lid 12. The outer upper surface of the flange 12a is rounded as shown in Figure 12 while the lower outer edge of the flange 12a is generally flat to mate with the gasket leg 16d when the lid is seated as shown in Figure 13. The juncture between the flange 12a and the remainder of the lid 12 on the inner surface of the lid is smoothly rounded as can be seen from Figure 12. Note also that the vertical thickness of the flange 12a is slightly greater than the wall thickness at the outer extremity of the flange. As seen from Figure 12, positioning the gasket on the lid flange causes the gasket leg 16d to move upwardly somewhat so that the walls 16b, 16c and 16d move closer to a U-shape. The gasket assumes this configuration where ever it can hang free on the lid 12. In other words, referring to Figure 1, the gasket would assume the position shown in Figure 12 throughout its periphery except that the gasket on the left end of the container will appear approximately as shown in Figure 5 and the gasket on the right end of the container in the area of the support actuator 18 will be as shown in Figure 10.

Operation

When the container is first placed in the autoclave, the actuator will be in the position shown in Figs. 1 and 10 holding the lid open and the expandable chamber 20 will be in the position shown in Figure 8. If the particular autoclave cycle being used includes one or more preliminary vacuum phases to withdraw air from the container, no movement of the actuator will occur, since the port 48 and the valve for the inflatable chamber 20 are open and not covered by the sleeve 50. Any pressure changes within the autoclave will be automatically applied to the interior of the chamber as well. When a high temperature sterilizing fluid such as gas or steam is applied to the autoclave, the fluid flows into the interior of the container and through the open lid into the interior of the basket through the ports 24, to displace the air and sterilize the container and the basket contents. Since the gasket 16 is positioned on the lid 12 relatively loosely, it has been found that the sterilizing fluid will also effectively sterilize the lower surfaces of the lid and the surfaces of the gasket.

The sterilizing environment applied to the container will of course also enter the chamber 20 through the ports 48. The elevated temperature of the fluid will cause the sleeve-like valve element 50 to shrink and cover the ports 48, as shown in Figure 9. The high temperature, high pressure fluid is thus captured in the chamber. No change however occurs in the volume of the chamber during the remainder of the sterilizing phase, since temperature and pressure surrounding the chamber is essentially the same as that within it. most autoclaves have some minor variations in temperatures and pressures during the sterilizing phase but such variations are not significant enough to cause the actuator to perform its actuating function. Thus, during the entire sterilizing phase, the lid of the container remains raised on one edge from the base such that fluid can flow freely into and out of the container. It is important that the lid be raised sufficiently to permit the sterilizing fluid to circulate freely and displace the air in the container. Preferably the lid should be raised at least a third of the height of a dome-shaped lid. It is also important that the circulation holes 24 in the basket be sized and spaced to permit the sterilizing fluid to displace the air in the basket. Condensate drains from the basket 22 through the holes in the bottom, and from the container through the drain holes 26 in the container base 14.

At the completion of the sterilizing phase of the cycle, there is an immediate pressure drop. Temperature also drops but this is much more slowly. As the pressure drops in the autoclave, the expandable chamber 20 expands due to the fact that the pressure of the steam captured within the chamber is greater than the pressure surrounding it. Thus, the bellows-like diaphragm 44 of the chamber 20 will move to the configuration shown in phantom lines in Figure 8 and shown in solid lines in Figure 3 and 7. Since the central wall 44h of the diaphragm 44 is engaging the outer edge of the lid or its gasket, 16, as shown in Figure 10, the actuator plate 28 is urged to pivot in a clockwise direction into the phantom line position shown in Figure 10, this position also being shown in Figure 3. The actuator moves because the resistance to movement provided by cantilever mounting arrangement is much less than that of the lid 12. Thus, as the actuator moves, its projections or posts 34 are withdrawn from beneath the lid, allowing the lid to fall. Note from Figure 8, that the wall 44h of the diaphragm 44 extends beyond the tip of the projections 34 when the bellows is fully expanded. This ensures that the lid will be released. With the lid released, the actuator will move back slightly somewhat towards the upper portion of the container lid, as shown in Figure 3, but this movement is somewhat limited while the expandable chamber is still expanded.

The lid falls into the proper position on the base and the gasket 16 assumes the approximate position shown in Figure 13. The sloping wall 14d helps guide the lid into the peripheral groove 15 and the surfaces of the groove are smoothly curved to facilitate proper positioning of the lid. Correspondingly, the extremely flexible and resilient flap 16f on the gasket ensures a proper seal on a reliable basis. In a gravity-type autoclave cycle wherein there is no final vacuum phase for withdrawing residual sterilizing environment from the autoclave, a vacuum is nevertheless formed within the container as the residual environment within the container cools and condenses, and as atmospheric pressure is introduced into the autoclave surrounding the container. The pressure differential between the interior and the exterior of the container may be quite small for a period of time in some situations such that it is important that the gasket be capable of preventing flow into the container at this time and the feathered edge of the gasket performs this function. At the same time, if the pressure within the container should be temporarily greater than the pressure on the outside of the container, the gasket feathered edge will readily permit flow out of the container, thus acting like a one-way valve. As the pressure on the interior of the container drops relative to the exterior atmospheric pressure, the lid is drawn more tightly against the base thus compressing the gasket more. This causes the bead 16e of the gasket to be further compressed between the lid and the base, becoming the primary seal for the container.

If the container is utilized in an autoclave providing a final vacuum phase, the residual environment in the autoclave is quickly withdrawn and the residual environment within the container is likewise withdrawn past the feathered edge of the gasket. When atmospheric pressure is introduced into the autoclave, the feathered edge of the gasket prevents flow into the container; and a quickly produced pressure differential between the interior and exterior of the container compresses the gasket greatly so that the bead 16e seals the container more tightly. Consequently, the container contents are sealed in essentially atmosphere free sterile environment, until the contents are to be used.

When the container is removed from the autoclave, the actuator 18 may be manually removed from the slot 32 in the container base and discarded. It is convenient to have a disposable type in a hospital environment, and the economics are such that this is a very practical approach. Alternatively, the actuator could be recycled by installing a new temperature responsive valve in the expandable chamber, or by employing a valve of a type that would recycle automatically. As one example the nipple 46 could be made as a separate component and be removably attached to the plate, and thus could be removed to permit replacement of the valve element 50 and then reinstalled. Such an approach might be most practical, if sterilization of the container and their contents is to be performed by specialists at a central location.

The container and its contents are then transported to a storage area or to the point where the contents are needed. In use, the container is typically moved to the general place of use, but the lid of the container is actually removed at a location somewhat remote from the actual operating or other use location in that the exterior of the container is contaminated during storage. When the lid is removed, the sterile basket on the interior protects the contents from falling dirt or other particles. The basket is carried to the actual location of use, and the cover on the basket is removed to provide access to the instruments or other items within the basket. This approach provides maximum sterility.

### Relief Valve

Because of the high vacuum within the container, it is impossible to remove the container lid without first relieving the vacuum. For this purpose, the relief valve 60 shown in Figures 1, 14, 15 and 16 is provided. As seen from Figure 1, the relief valve is located in the top wall of the lid 12; however, it should be recognized that such a valve can be placed in other locations as well. Referring to Figure 14, the valve is made of flexible resilient material as a one-piece member except for an inner filter 62. The valve includes a generally tubular projection or plug 64 which is open on its lower end and enclosed by an enlarged resilient flange 66 on its upper or outer end. A passage 68 through the projection 64 opens to a port 70 in a side wall of the projection immediately beneath the flange 66.

In use, the projection 64 is inserted through an opening in the lid 12. This operation is performed with the rigid, metal foam filter removed. The filter 62 is then installed in the lower end of the plug 64 as shown in Figure 15. This not only secures the filter within the plug extending across the passage, but also helps pull the valve in a sealed condition in combination with a ring 65 on the exterior of the plug. The normal position of the valve when the container is not under vacuum is as shown in Figure 15 with the annular edge 66a of the flange 66 against the outer surface of the lid thus preventing flow into the container through the port 70 and passage 68.

When the container lid 12 moves to its closed position, the valve 60 prevents air flow into the container; and as a vacuum is formed within the container, the exterior pressure forces most of the lower surface of the valve flange 66 against the lid. The annular relief groove 69 in the upper surface of the flange 66 and the annular groove 67 in the lower surface adjacent the plug 64 enable the flange to flatten readily. In addition, the thin upper wall 66b of the flange covering the end of the passage 68 is drawn inwardly because of the vacuum. This provides visible indication to an observer that a vacuum condition exists in a particular container.

When the vacuum is to be released to enable the container lid to be withdrawn, a tab 74 is manually pulled to lift the edge of the flange 66 away from the lid so that air may flow into the port 70 and through the passage 68 in the valve. If desired, the tab may be hooked on a tab holder 76 as shown in Figure 16. This may be convenient in that the filter 60 is so fine that it will take several seconds for the pressure to equalize in a large container. All of the air entering the container must of course pass through the filter 62. Consequently, even though the entering air has not been subjected to high temperature sterilization, a high percentage of the dust, lint and other particles within the air are removed as the air passes through the filter. Once the container interior and exterior equalize, the lid can be lifted off of the base to provide access to the inner basket.

### Lid Support

Although the container 10 is constructed to withstand a high vacuum, it has been found desirable to provide further mechanical support for large containers. A preferred approach for providing such support is illustrated in Figure 17. The basket cover 23, which is supported on its periphery by the basket base 22, is dimensioned so that its upper surface mates with the lower surface of the lid 12; and normally with a container lid tightly closed on a container base, the lid would be slightly spaced from the inner basket, however, if an overstressed condition should occur, such that the lid 12 should begin to buckle, it will engage the upper surface of the basket cover 23 to be supported thereby.

Further, with particularly large containers, the lower portion of the inner basket 22 is provided with an upwardly extending cone-shaped projection 78 that terminates near the cover 23 of the basket. The periphery of the basket cover 23 rests on the basket base 22, but it also engages or comes close to engaging the upper end of the support cone. The container lid 12 is formed with recesses that are complementarily received in the basket cover. Thus, in the overstressed situation the basket cover will rest upon the upper end of the support cone 78 on the basket base, thus preventing collapse of the lid. Of course, a suitable support may be provided as a separate element, or attached to either the container lid or base, and used without a basket; or used with a modified basket which would fit with a support.

Although the container is primarily designed for use with a steam or gas autoclave sterilizing cycle, it should be understood that it is also very useful with other sterilizing techniques. With microwave sterilizing, the container lid may be positioned on the container base in a lightly closed condition. As the contents are heated, any pressure increase within the container may vent from the container past the flexible gasket. When the container cools, a vacuum will be formed in the container, automatically pulling the lid more tightly closed on the container base.

With radiation sterilizing, which does not rely on heat, the container lid is placed on the base in a lightly closed position, and the container is then subjected to a vacuum to withdraw air from the container past the flexible gasket. When pressure around the container is again allowed to increase, the lid will be tightly compressed on the base, since the gasket will prevent a pressure increase in the container. The container is then subjected to radiation, leaving the container contents sterilized and sealed in an essentially atmosphere free environment.

The container may be used for a wide variety of items in addition to surgical instruments. If it is used solely for towels, bandages, and other such somewhat bulky items, it may be convenient to invert the container so that the lid becomes the base, and not use the basket. The side walls of the inverted lid will hold items like towels more easily than will a flat base. The expandable chamber actuator would function in the same manner as described above. Once the container is closed, it could of course be returned to original position for storage and ease of handling. In the inverted position there would be no provision for drainage with the container illustrated; but there would be no drainage with towels. If desired the relief valve in the inverted lid may be modified to be open during the sterilizing phase and then automatically closed in response to temperature. In addition to being a drain for condensate, a valve of this type would more importantly allow air to drain from the container as the steam or other sterilizing fluid is applied.

**Claims**

1. A device responsive to pressure and temperature and comprising an expandable chamber (20) impart defined by a flexible diaphragm (44) and communicating with the atmosphere via a port (48) which can be closed by a temperature responsive valve element (50) to capture a quantity of fluid in the chamber (20), which fluid is to expand the chamber (20) and thereby flex the diaphragm (44) when the pressure outside the diaphragm (44) becomes less than the pressure inside the chamber (20), characterised in that the device (18) includes a thin but relatively stiff plastic plate (28) to which the diaphragm (44) is secured to define the chamber (20) between the plate (28) and the diaphragm (44), in that said port (48) is formed by a passage in said plate (28), and in that said diaphragm (44) is in the form of a thin plastic, bellows-like element.

2. A device according to claim 1, in which the plate (28) is mounted on one (14) of two members (12 and 14) which cooperate with one another to form a container (10) and includes means (34) formed thereon to cooperate with the other member (12) to prop the container (10) open, the arrangement being such that expansion of fluid trapped in the chamber (20) is utilized to displace the plate (28) and therewith the cooperating means (34) to permit the container to close.

3. A device according to claim 2, in which the cooperating means (34) comprises one or more projections extending outwardly from the plate (28) to engage said other container member (12) and hold the container (10) open.

4. A device according to claim 2 or 3, which is so arranged as to engage said other container member (12) when the chamber (20) expands, so causing the plate (28) and the cooperating means (34) thereon to be pushed away from said other container member (12).

5. A device according to claims 3 and 4, in which the diaphragm (44) includes a portion (44h) which extends beyond the projections (34) to enable it to engage said other container member (12) when the chamber (20) is expanded.

6. A device according to any of claims 2 to 5, in which the plate (28) has a tab (30) which fits in a slot (32) in said one member (14) to mount the plate (28) on the latter.

7. A device according to any of claims 1 to 6, in which a nipple (46) is formed on said plate (28) and extends into the chamber (20), said passage (48) being formed in the nipple (46), and in which said valve element (50) comprises a heat shrinkable valve element surrounding the nipple (46), said valve element (50) shrinking to close the passage (48) when it is exposed to a predetermined temperature.

8. A device according to claim 7, in which the passage (48) is located in a side wall of the nipple (46) at an angle such that the passage may be formed during a moulding operation in which the plate (28) is made.

9. Apparatus for containing items to be sterilized and subsequently stored, comprising a container (10) formed by a base (14) and a lid (12) cooperating with the base, and a device (18) mounted on a peripheral flange (14e) of the base (14) and including an element (34) for propping the lid (12) in an open position relative to the base (14) and flexible means (44) in part defining an expandable chamber (20) effective upon expansion so that the flexible means (44) displaces the propping element (34), allowing the lid to move to a closed position relative to the base, characterised in that the device (18) includes a support member (28) whose lower end (30) is connected to the base flange (14e) and whose upper position has at least one projection (34) thereon, said projection being arranged to be normally beneath the rim (12a) of the lid (12) so as to institute said element for propping the lid open, in that said support member (28) is so constructed as to bias the upper position thereof towards the propped lid (12) and so maintain the projection (34) beneath the lid rim (12a), and in that the expandable chamber (20) is defined between the support member (28) and the flexible means (44) which is carried thereby, such that the lid is engaged by the device (18) upon expansion of the chamber (20) to displace the upper position of the support member (28) away from lid and thus withdraw the projections (34) from beneath the lid rim (12a), thereby allowing the lid (12) to fall to a closed position relative to the base (14).

## Patentansprüche

1. Druck- und temperaturempfindliche Vorrichtung mit einer expandierbaren Kammer (20), die zum Teil durch eine flexible Membrane (44) festgelegt ist und die mit der Atmosphäre über eine Pforte (48) verbunden ist, welche durch ein auf die Temperatur ansprechendes Ventilelement (50) verschließbar ist, um eine Fluidmenge in der Kammer (20) einzuschließen, wobei das Fluid die Kammer (20) vergrößern und dabei die Membrane (33) auswölben wird, wenn der Druck außerhalb der Membrane (44) geringer wird als der Druck innerhalb der Kammer (20), dadurch gekennzeichnet, daß die Vorrichtung (18) eine dünne, aber relativ steife Kunststoffplatte (28) aufweist, an welcher die Membrane (44) zur Festlegung der Kammer (20) zwischen der Platte (28) und der Membrane (44) befestigt ist, daß die Pforte (48) von einem Durchlaß in der Platte (28) gebildet ist und daß die Membrane (44) die Form eines dünnen, aus Kunststoff bestehenden balgartigen Elementes aufweist.

2. Vorrichtung nach Anspruch 1, bei welcher die Platte (28) an einem (14) von zwei Teilen (12 und 14), welche miteinander wirken, um einen Behälter (10) zu bilden, befestigt ist und an ihr ausgebildete Mittel (34) aufweist, welche mit dem anderen Teil (12) zusammenwirken, um den Behälter (10) offenzuhalten, wobei die Anordnung so getroffen ist, daß eine Expansion des in der Kammer (20) eingeschlossenen Fluids verwendet wird, um die Platte (28) und mit ihr die zusammenarbeitenden Mittel (34) zu verstellen, um das Schließen des Behälters zu gestatten.

3. Vorrichtung nach Anspruch 2, bei welcher die zusammenwirkenden Mittel (34) einen oder mehrere Vorsprünge aufweisen, die nach außen von der Platte (28) abstehen, um den anderen Behälterteil (12) zu berühren und den Behälter (10) offenzuhalten.

4. Vorrichtung nach Anspruch 2 oder 3, welche so eingerichtet ist, daß sie beim Expandieren der Kammer (20) mit dem anderen Behälterteil (12) berührt und so die Platte (28) und die zusammenwirkenden Mittel (34) auf dieser veranlaßt, von dem anderen Behälterteil (12) weggedrückt zu werden.

5. Vorrichtung nach den Ansprüchen 3 und 4, bei welcher die Membran (44) einen Abschnitt (44h) beinhaltet, welcher über die Vorsprünge (34) hinaus vorsteht, damit er den anderen Behälterteil (12) berühren kann, wenn die Kammer (20) expandiert ist.

6. Vorrichtung nach einem der Ansprüche 2 bis 5, bei welcher die Platte (28) einen Lappen (30) aufweist, der in einen Schlitz (32) in dem einen Teil (14) hineinpaßt, um die Platte (28) auf letzterem anzubringen.

7. Vorrichtung nach einem der Ansprüche 1 bis 6, bei welcher ein Wippel (46) auf der Platte (28) ausgebildet ist und in die Kammer (20) hineinragt, wobei der Durchgang (48) in dem Nippel (46) ausgebildet ist und bei welcher das Ventilelement (50) ein wärmeschrumpfbares Ventilelement auf-

weist, das den Nippel (46) umgibt, wobei ein Schrumpfen des Ventilelements (50), wenn es einer vorbestimmten Temperatur ausgesetzt wird, den Durchgang (48) verschließt.

8. Vorrichtung nach Anspruch 7, bei welcher der Durchgang (48) in einer Seitenwand des Nippels (46) unter einem solchen Winkel angeordnet ist, daß der Durchgang während eines Spritzgußvorganges, bei welchem die Platte (28) hergestellt wird, ausgeformt werden kann.

9. Gerät zum Aufnehmen von Gegenständen, die sterilisiert und darauffolgend gelagert werden sollen, bestehend aus einem Behälter (10), der gebildet wird von einem Unterteil (14) und einem mit dem Unterteil zusammenwirkenden Deckel (12), und einer Vorrichtung (18), die an einem Umfangsflansch (14e) des Unterteiles (14) befestigt ist und ein Element (34) aufweist zum Abstützen des Deckels (12) in einer relativ zum Unterteil (14) offenen Stellung, und einem flexiblen Mittel (44), welches zum Teil eine vergrößerbare Kammer (20) festlegt, die bei ihrer Expansion wirksam wird, so daß das flexible Mittel (44) das Abstützelement (34) verstellt, wodurch der Deckel sich in eine relativ zum Unterteil geschlossene Stellung bewegen kann, dadurch gekennzeichnet, daß die Vorrichtung (18) ein Stützglied (28) aufweist, dessen unteres Ende (30) mit dem Flansch (14e) des Unterteiles verbunden ist und dessen oberer Bereich zumindest einen Vorsprung (34) aufweist, der so angeordnet ist, daß er sich für gewöhnlich unterhalb des Randes (12a) des Dekkels (12) befindet, um das Abstützelement für das Offenhalten des Deckels abzugeben, daß das Stützglied (28) so ausgebildet ist, daß sein oberer Abschnitt gegen den abgestützten Deckel (12) geneigt ist und dadurch der Vorsprung (34) unterhalb des Deckelrandes (12a) gehalten wird, und daß die expandierbare Kammer (20) zwischen dem Stützglied (28) und dem von ihm getragenen flexiblen Mittel (44) festgelegt ist, so daß bei der Expansion der Kammer (20) der Deckel von der Vorrichtung (18) berührt wird, um den oberen Abschnitt des Stützgliedes (28) von dem Deckel wegzurücken und solcherart den Vorsprung (34) von unterhalb des Deckelrandes (12a) zurückzuziehen, wodurch der Deckel (12) in eine Schließstellung relativ zum Unterteil (14) fallen kann.

## Revendications

1. Dispositif sensible à la pression et à la température et comportant une chambre expansible (20) délimitée en partie par un diaphragme souple (44) et communiquant avec l'atmosphère par l'intermédiaire d'un orifice (48) qui peut être fermé par un élément d'obturation (50) réagissant à la température afin d'emprisonner une certaine quantité de fluide dans la chambre (20), lequel fluide est destiné à provoquer l'expansion de la chambre (20) et à imposer de la sorte une flexion au diaphragme (44) lorsque la pression régnant à l'extérieur du diaphragme (44) devient plus faible que la pression régnant à l'intérieur de la chambre (20), caractérisé par le fait que le dispositif (18) présente une plaque (28) en matière plastique mince mais relativement rigide, à laquelle le diaphragme (44) est fixé pour délimiter la chambre (20) entre la plaque (28) et le diaphragme (44); par le fait que ledit orifice (48) est formé par un passage dans ladite plaque (28); et par le fait que ledit diaphragme (44) se présente sous la forme d'une mince pièce en matière plastique du type soufflet.

2. Dispositif selon la revendication 1, dans lequel la plaque (28) est montée sur l'un (14) de deux éléments (12 et 14) qui coopèrent mutuellement pour former un réceptacle (10), et comporte des moyens solidaires (34) pour coopérer avec l'autre élément (12) afin de maintenir fermement le réceptacle (10) ouvert, l'agencement étant tel qu'une expansion du fluide emprisonné dans la chambre (20) est utilisée pour déplacer la plaque (28) et avec elle les moyens associés (34), afin de permettre la fermeture du réceptacle.

3. Dispositif selon la revendication 2, dans lequel les moyens associés (34) consistent en une ou plusieurs saillies s'étendant vers l'extérieur au-delà de la plaque (28) pour venir en prise avec ledit autre élément (12) du réceptacle et maintenir ce réceptacle (10) ouvert.

4. Dispositif selon la revendication 2 ou 3, qui est agencé de façon à venir en prise avec ledit autre élément (12) du réceptacle lorsque la chambre (20) accuse une expansion, obligeant ainsi la plaque (28) et les moyens associés (34) qui s'y trouvent à être poussés à l'écart dudit autre élément (12) du réceptacle.

5. Dispositif selon les revendications 3 et 4, dans lequel le diaphragme (44) comporte une région (44h) qui s'étend au-delà des saillies (34) pour lui permettre de venir en prise avec ledit autre élément (12) du réceptacle lorsque la chambre (20) accuse une expansion.

6. Dispositif selon l'une quelconque des revendications 2 à 5, dans lequel la plaque (28) possède une languette (30) qui est ajustée dans une fente (32) pratiquée dans ledit élément (14), afin de monter la plaque (28) sur ce dernier.

7. Dispositif selon l'une quelconque des revendications 1 à 6, dans lequel un raccord (46) est formé sur ladite plaque (28) et s'étend dans la chambre (20), ledit passage (48) étant ménagé dans le raccord (46); et dans lequel ledit élément d'obturation (50) consiste en un élément obturateur thermorétractable entourant le raccord (46), ledit élément d'obturation (50) se rétractant pour fermer le passage (48) lorsqu'il est exposé à une température prédéterminée.

8. Dispositif selon la revendication 7, dans lequel le passage (48) est situé dans une paroi latérale du raccord (46), à un angle tel que ce passage puisse être formé lors d'une opération de moulage au cours de laquelle la plaque (28) est fabriquée.

9. Appareil pour renfermer des objets devant être stérilisés puis stockés, comprenant un réceptacle (10) formé par une base (14) et un couvercle (12) coopérant avec cette base, ainsi qu'un dispositif (18) installé sur une aile périphérique (14e) de la base (14) et renfermant un élément (34) pour maintenir fermement le couvercle (12) dans une position ouverte par rapport à la base (14) et un moyen souple (44) délimitant en partie une chambre expansible (20) exerçant son action lors de l'expansion de façon que le moyen souple (44) déplacé l'élément de maintien (34), permettant au couvercle de se mouvoir jusqu'à une position fermée par rapport à la base, caractérisé par le fait que le dispositif (18) comporte une pièce de support (28) dont l'extrémité inférieure (30) est reliée à l'aile (14e) de la base et dont la région supérieure possède au moins une saillie (34), ladite saillie étant agencée de façon à se trouver normalement au-dessous du rebord (12a) du couvercle (12) afin de constituer ledit élément pour maintenir fermement le couvercle ouvert; par le fait que ladite pièce de support (28) est réalisée de manière à pousser sa région supérieure en direction du couvercle maintenu (12) et à retenir ainsi la saillie (34) au-dessous du rebord (12a) du couvercle; et par le fait que la chambre expansible (20) est délimitée entre la pièce de support (28) et le moyen souple (44) supporté par cette dernière, de telle sorte que le dispositif (18) vienne en prise avec le couvercle, lors de l'expansion de la chambre (20), pour déplacer la région supérieure de la pièce de support (28) à l'écart dudit couvercle et pour dégager ainsi la saillie (34) de son emplacement sousjacent au rebord (12a) du couvercle, en permettant alors une chute du couvercle (12) jusqu'à une position fermée par rapport à la base (14).

*Fig. 1*

*Fig. 2*

*Fig. 3*

1

*Fig. 4*

*Fig. 5*

*Fig. 6*

*Fig. 7*

Fig. 8

Fig. 9

Fig. 10

Fig. 11

Fig. 12

Fig. 13

Fig. 14

Fig. 15

Fig. 16

Fig. 15a

Fig. 17

4